(19) Europäisches Patentamt
European Patent Office
Office européen des brevets

(11) Veröffentlichungsnummer: **0 002 219**
**B1**

(12) # EUROPÄISCHE PATENTSCHRIFT

(21) Anmeldenummer: 78101401.4

(22) Anmeldetag: 18.11.78

(51) Int. Cl.$^3$: A 61 K 31/265, C 07 C 147/00, C 07 C 149/20, C 07 C 153/07

(54) **Arzneimittel mit schwerfelhaltigen Estern und Verfahren zu ihrer Herstellung**

(30) Priorität: 02.12.77 DE 2753768
03.06.78 DE 2824386

(43) Veröffentlichungstag der Anmeldung:
13.06.79 Patentblatt 79/12

(45) Bekanntmachung des Hinweises auf die Patenterteilung:
10.12.80 Patentblatt 80/25

(84) Benannte Vertragsstaaten:
BE CH DE FR GB NL SE

(56) Entgegenhaltungen:
DE - A - 2 629 599
DE - A - 2 642 016
FR - M - 5 551

CHEMICAL ABSTRACTS, Vol. 63, Nr. 15391c, 1965,
Columbus, Ohio, USA

CHEMICAL ABSTRACTS, Vol. 74, Nr. 110038d, 1971
Columbus, Ohio, USA
H. SPRINCE et al., "3-Mercaptopropionic acid: convulsant and lethal properties compared with other sulfur-convulsants; protection therefrom", Seite 245

(73) Patentinhaber: Troponwerke GmbH & Co. KG
Berliner Strasse 156
D - 5000 Köln 80 (DE)

(72) Erfinder: Opitz, Wolfgang, Dr.
Silesiusstrasse 80
D - 5000 Köln 80 (DE)
Etschenberg, Eugen, Dr.
Virchowstrasse 20
D - 5000 Köln 41 (DE)
Dell, Hans-Dieter, Dr.
Kalmüntener Strasse 5
D - 5060 Berg. Gladbach 2 (DE)
Jacobi, Haireddin, Dr.
Finkenweg 2
D - 5652 Leichlingen (DE)

(74) Vertreter: Heimbach, Karl Josef, Dr., et al
BAYER AG c/o Zentralbereich Patente Marken und Lizenzen
D - 5090 Leverkusen 1, Bayerwerk (DE)

EP 0 002 219 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

# Arzneimittel mit schwefelhaltigen Estern und Verfahren zu ihrer Herstellung

Die vorliegende Erfindung betrifft neue Arzneimittel enthaltend teilweise bekannte schwefelhaltige Ester, insbesondere Arzneimittel mit antiphlogistischer Wirkung und Verfahren zu ihrer Herstellung.

Die Verwendung der erfindungsgemaßen schwefelhaltigen Ester als Arzneimittel ist noch nicht bekannt.

Es wurde gefunden, daß die schwefelhaltigen Ester der allgemeinen Formel I

$$X{-}Y{-}\overset{\displaystyle O}{\overset{\|}{C}}{-}Z \qquad (I)$$

in welcher

X für ein Wasserstoffatom, eine Alkylgruppe, eine Alkoxygruppe, eine Alkylthiogruppe, eine Alkylsulfinyl- oder Alkylsulfonylgruppe oder für eine Hydroxy- oder eine Mercaptogruppe steht,

Y für die Gruppierung

$$-CH_2-\underset{\displaystyle R}{\underset{|}{CH}}- \quad \text{oder} \quad -CH{=}\underset{\displaystyle R}{\underset{|}{C}}-$$

wobei R für ein Wasserstoffatom oder eine Alkylgruppe steht, und

Z für eine Alkylthiogruppe steht,

eine vorteilhafte entzündungshemmende Wirkung aufweisen.

Einige der erfindungsgemäßen beanspruchten Wirkstoffe der allgemeinen Formel I sind bekannt und zum Teil im Handel erhältlich. Eine therapeutische Wirkung dieser Verbindungen ist bisher noch nicht bekannt geworden.

Für einige chemische ähnliche Verbindungen sind bereits therapeutische oder pharmakologische Wirkungen beschrieben. So wird z. B. in Chemical Abstracts, Vol 63, Nr. 15391c, 1965, die 3-(Undecylthio)propionsäure und die 3-(Dodecylthio)propionsäure als Arzneimittel mit Antitumorwirkung beschrieben. Ebenso ist in Chemical Abstract, Vol. 74, Nr. 110038d, 1971, die 3-Mercaptopropionsäure als convulsiv beschrieben. Aus dem französischen Patent M5551 sind weiterhin Propionsäurederivate, die am Alkylrest eine schwefelhaltige Gruppierung tragen, als Arzneimittel bekannt. Auch in der Deutschen Anmeldung Nr. 26 29 599 sind Derivative von ungesättigten Carbonsäuren und Carbonsäureestern beschrieben, deren Kohlenstoffkette durch Thioalkyl- und Thioalkenylgruppen substituiert ist. Sie werden als Wirkstoffe in kosmetischen Mitteln angewandt. Keins dieser bekannten Carbonsäurederivate enthält eine Thioestergruppierung und für keins dieser Derivate ist eine entzündungshemmende Wirkung beschrieben.

Die Darstellung der erfindungsgemäßen Verbindungen erfolgte überwiegend gemäß nachfolgendem Reaktionsschema, wie an einem Beispiel aufgezeigt sei:

$$CH_2{=}CH{-}\overset{\displaystyle O}{\overset{\|}{C}}{-}Cl + 2\,CH_3SH \xrightarrow{-HCl} CH_3SCH_2CH_2{-}COSCH_3 \xrightarrow{+H_2O_2}$$

$$CH_3SOCH_2CH_2COSCH_3 \xrightarrow{H_2O_2} CH_3SO_2CH_2CH_2COSCH_3$$

Je nach Konzentration des Oxidationsmittels und je nach angewandter Reaktionsdauer können die einzelnen Oxidationsstufen abgefangen werden, bzw. es entstehen Gemische, die chromatographisch aufgetrennt werden können, wie im Versuchsteil im einzelnen beschrieben ist.

Überraschenderweise zeigen die erfindungsgemäßen schwefelhaltigen Ester bei sehr guter entzündungshemmender Wirkung eine wesentlich größere therapeutische Breite als die bisher bekannten Verbindungen auf diesem Gebiet. Außerdem zeigen sie geringere Nebenwirkungen, wie z.B. Sodbrennen, Bildung von Ulcera, Darmblutungen usw. Die erfindungsgemäßen Verbindungen stellen somit eine Bereicherung der Pharmazie dar.

Als Beispiele für die erfindungsgemäß verwendbaren Verbindungen seien im einzelnen genannt:

S-Methylmethacrylthioat ($CH_2{=}C(CH_3)COSCH_3$)

S-Methylcrotonylthioat ($CH_3CH{=}CH{-}COSCH_3$)

S-Äthylacrylthioat ($CH_2=CH-COSC_2H_5$)

S-Äthyl-3-äthylthiopropionylthioat ($C_2H_5SCH_2CH_2COSC_2H_5$)

S-Methyl-3-methylthio-2-propylcarbonylthioat ($CH_3SCH_2CH(CH_3)COSCH_3$

S-Methyl-3-methylthiopropianthioat ($CH_3S-CH_2-CH_2-COSCH_3$)

S-Propyl-3-propylthiopropionthioat ($C_3H_7SCH_2CH_2COSC_3H_7$)

S-Isopropyl-3-isopropylthiopropionthioat ($CH_3CH(CH_3)SCH_2CH_2-COSCH(CH_3)CH_3$)

S-Butyl-3-butylthiopropionthioat ($C_4H_9SCH_2CH_2COSC_4H_9$)

S-sek.Butyl-3-sek.butylthiopropionthioat ($CH_3CH_2CH(CH_3)SCH_2CH_2COSCH(CH_3)CH_2CH_3$)

S-tert.Butyl-3-tert.butylthiopropionthioat ($(CH_3)_3CSCH_2CH_2COSC(CH_3)_3$)

S-Isobutyl-3-isobutylthiopropionthioat ($CH_3CH(CH_3)CH_2SCH_2CH_2COSCH_2CH(CH_3)_2$)

S-Butyl-3-butylsulfinylpropionthioat ($C_4H_9SOCH_2CH_2COSC_4H_9$)

S-sek.Butyl-3-sek.butylsulfinylpropionthioat ($CH_3CH_2CH(CH_3)SOCH_2CH_2COSCH(CH_3)CH_2CH_3$)

S-Isopropyl-3-isopropylsulfinylpropionthioat ($(CH_3)_2CHSOCH_2CH_2COSCH(CH_3)_2$)

S-Propyl-3-propylsulfinylpropionthioat ($C_3H_7SOCH_2CH_2COSC_3H_7$)

S-Äthyl-3-äthylsulfinylpropionthioat ($C_2H_5SOCH_2CH_2COSC_2H_5$)

S-tert.Butyl-3-tert.butylsulfinylpropionthioat ($(CH_3)_3CSOCH_2CH_2COSC(CH_3)_3$)

S-Äthyl-3-äthylsulfonylpropionthioat ($C_2H_5SO_2CH_2CH_2COSC_2H_5$)

S-sek.Butyl-3-sek.butyl-sulfonyl-propionthioat ($CH_3CH_2CH(CH_3)SO_2=CH_2CH_2COSCH(CH_3)CH_2CH_3$)

S-Isobutyl-3-isobutylsulfonylpropionthioat ($(CH_3)_2CHCH_2SO_2CH_2CH_2COSCH_2CH(CH_3)_2$)

S-tert.butylsulfonylpropianthioat ($(CH_3)_3CSO_2CH_2CH_2COSC(CH_3)_3$)

S-Butyl-3-butylsulfonylpropianthioat ($C_4H_9SO_2CH_2CH_2COSC_4H_9$)

S-Isopropyl-3-isopropylsulfonylpropianthioat ($(CH_3)_2CHSO_2CH_2CH_2COSCH(CH_3)_2$)

S-Propyl-3-propylsulfonylpropianthioat ($C_3H_7SO_2CH_2CH_2COSCH_3H_7$)

S-Methyl-3-methylsulfonylpropianthioat ($CH_3SO_2CH_2CH_2COSCH_3$)

Die pharmakologische Wirkung sei beispielhaft anhand des S-Methyl-3-methylthiopropionthioats (nachfolgend als A bezeichnet) demonstriert.

Die Substanz A besitzt eine sehr ausgeprägte antiphlogistische Wirkung und ist in hervorragendem Maße geeignet für die parenterale Anwendung. Die entzündungshemmende Wirkung wurde an dem durch Kaolinapplikation ausgelösten Pfotenödem der Ratte nachgewiesen. Der Wert für die $DE_{50}$ betrug 4,85 $\mu$mol/kg nach i.m.-Injektion.

Dieser Wert zeigt deutlich, auch im Vergleich mit den bei gleicher Methode ermittelten $DE_{50}$-Werten für bekannte handelsübliche Antiphlogistika (s. Tabelle 1), daß die Verbindung A die experimentelle Entzündung in starkem Maße hemmt.

Es is hinreichend bekannt, daß nahezu alle nichtsteroidalen Antiphlogistika bei Mensch und Tier zu Schäden der Schleimhaut des Gastrointestinaltraktes führen. Deshalb wurde geprüft, ob die Verbindung A nach i.m.-Anwendung eine ulcerogene Wirkung besitzt.

Bei der Ratte wurde eine $DE_{50}$ von 256,41 $\mu$mol/kg gefunden. Der Dosenabstand zwischen beiden $DE_{50}$-Werten läßt schon erkennen, daß, gemassen an diesen beiden Kriterien, für die Verbindung A eine große therapeutische Breite besteht.

Darüber hinaus ist A auch an anderen Entzündungsmodellen wirksam. Nachstehende $DE_{50}$ Werte

werden ermittel (mg/kg; i.m) Formalinödem 5,4, Eiweißödem 6,3, Hefeödem 27,4, Cancanavalinödem 3,4, Trypsinödem 2,0.

Diese Aussagen können noch verstärkt werden durch die Bestimmung der akuten Toxizität bei Ratten nach i.m-Injektion von Verbindung A.

Die $DL_{50}$ betrug 4.587 μmol/kg.

Der große Abstand zwischen diesem Wert und der $DE_{50}$ am Kaolinödem unterstreicht die unerwartete günstige therapeutische Breite für die Verbindung A. Die vorstehenden Ergebnisse sind in der Tabelle den entsprechenden Werten von Flufenaminsäure, Phenylbutazon und Indometacin gegenübergestellt. Der Vergleich der Zahlen in den beiden letzten Kolumnen der Tabelle lassen die herausragende Stellung der Verbindung A in Bezug auf ihre therapeutische Breite erkennen.

TABELLE 1

Vergleich der therapeutischen Breite

Ratte

| Substanz | Kaolinodem $DE_{50}$ µmol/kg | Magenulcus $DE_{50}$ µmol/kg | $DL_{50}$ µmol/kg | Applikat. art | therapeutische Breite: $\frac{DE_{50}\ Ulcus}{DL_{50}\ Kaolinodem}$ | therapeutische Breite: $\frac{DL_{50}}{DL_{50}\ Kaolinodem}$ |
|---|---|---|---|---|---|---|
| S-Methyl-3-methyl-thiopropionthioat | 4.85 5.76* 3.38 | 256.41 429.18 172.41 | 4587 4950 4255 | i.m. | 52.86 | 945.77 |
| Flufenaminsaure | 36.2 50.9 23.9 | 55.1 78.7 32.7 | 2037 3065 1632 | oral | 1.52 | 56.27 |
| Phenylbutazon | 122 167.4 92.8 | 181.6 234.9 145.0 | 3217 4803 2317 | oral | 1.48 | 26.36 |
| Indometacin | 2.9 3.9 2.3 | 11.1 21.0 6.4 | 68 85 55 | oral | 3.81 | 23.44 |

*) Angaben bedeuten Konfidenzbereiche mit einer Irrtumswahrscheinlichkeit von 5 %.

Über die für Verbindung A beschriebenen Effekte hinaus konnte auch nachgewiesen werden, daß die anderen Derivate (s. Tabelle 2) ebenfalls stark entzündungshemmend wirksam sind. In der Tabelle 2 sind die Grenz-Dosen aufgeführt, die nich eine eindeutig nachweisbare Beeinflussung des Pfotenödems nach Kaolinapplikation bewirken.

TABELLE 2

Antiphlogistische Wirkung
Ratte, Kaolinödem, i.m., n = 10/Dosengruppe

| Verbindung | Dosis mg/kg | Hemmung in % der Kontrollen (DE$_{50}$ in mg/kg |
|---|---|---|
| $C_2H_5SCH_2CH_2COSC_2H_5$ | 2.50 | 58.2 (3,75) |
| $CH_3CH{=}CHCOSCH_3$ | 12.50 | 16.0 |
| $CH_2{=}C(CH_3)COSCH_3$ | 5.00 | 21.8 |
| $CH_3SOCH_2CH_2COSCH_3$ | 3.12 | 10.2 (1,8) |
| $CH_3SCH_2CH(CH_3)COSCH_3$ | 0.65 | 16.6 (4,5) |
| $CH_2{=}CHCOSC_2H_5$ | 0.32 | 18.5 (1,5) |
| $(CH_3)_3CSCH_2CH_2COSC(CH_3)_3$ | 12,5 | 23,3 |
| $(CH_3)_2CHSCH_2CH_2COSCH(CH_3)_2$ | 25 | 63,2 |
| $C_3H_7SCH_2CH_2COSC_3H_7$ | 25 | 68,5 |
| $C_4H_9SCH_2CH_2COSC_4H_9$ | 50 | 77,2 |
| $CH_3CH_2CH(CH_3)SCH_2CH_2COSCH(CH_3)CH_2CH_3$ | 50 | 72 |
| $(CH_3)_2CHCH_2SCH_2CH_2COSCH_2CH(CH_3)_2$ | 50 | 77,2 |
| $(CH_3)_2CHSOCH_2CH_2COSCH(CH_3)_2$ | 12,5 | 22,7 |
| $(CH_3)_2CHCH_2SOCH_2CH_2COSCH_2CH(CH_3)_2$ | 12,5 | 17,9 |
| $CH_3CH_2CH(CH_3)SO_2CH_2CH_2COSCH(CH_3)CH_2CH_3$ | 12,5 | 33 |
| $(CH_3)_2CHCH_2SO_2CH_2CH_2COSCH_2CH(CH_3)_2$ | 12,5 | 42 |
| $C_3H_7SOCH_2CH_2COSC_3H_7$ | 100 | 73 |
| $CH_3CH_2CH(CH_3)SOCH_2CH_2COSCH(CH_3)CH_2CH_3$ | 100 | 90,5 |
| $C_4H_9SOCH_2CH_2COSC_4H_9$ | 100 | 73 |

Von besonderen Interesse ist die Verwendung von Verbindungen der allgemeinen Formel (I) in welcher

X für Wasserstoff, Alkyl, Alkoxy, Alkylthio, Alkylsulfinyl, Alkylsulfonyl oder eine Mercaptogruppe steht, wobei die vorgenannten Alkyl- und Alkoxygruppen jeweils 1 bis 4 Kohlenstoffatome enthalten;

Y für die Gruppierung

$—CH_2—CH—$ oder $—CH{=}C—$
| |
R R

steht, wobei R Wasserstoff oder Alkyl mit 1 bis 4 Kohlenstoffatomen bedeutet und Z für Alkylthio mit 1 bis 4 Kohlenstoffatomen im Alkylrest steht.

Zur vorliegenden Erfindung gehören pharmazeutische Zubereitungen, die neben nichttoxischen, inerten pharmazeutisch geeigneten Tägerstoffen einen oder mehrere erfindungsgmäße Wirkstoffe enthalten oder die aus einem oder mehreren erfindungsgemäßen Wirkstoffen bestehen sowie Verfahren zur Herstellung dieser Zubereitungen.

Zur vorliegenden Erfindung gehören auch pharmazeutische Zubereitungen in Dosierungseinheiten. Dies bedeutet, daß die Zubereitungen in Form einzelner Teile, z.B. Tabletten, Dragees, Kapseln, Pillen, Suppositorien und Ampullen vorliegen, deren Wirkstoffgehalt einem Bruchteil oder einem Vielfachen einer Einzeldosis entsprechen. Die Dosierungseinheiten können z.B. 1, 2, 3 oder 4 Einzeldosen oder 1/2, 1/3 oder 1/4 einer Einzeldosis enthalten. Eine Einzeldosis enthält vorzugsweise die Menge Wirkstoff, die bei einer Applikation verabreicht wird und die gewöhnlich einer ganzen, einer halben oder einem Drittel oder einem Viertel einer Tagesdosis entspricht.

Unter nichttoxischen, inerten pharmazeutisch geeigneten Trägerstoffen sind feste, halbfeste oder flüssige Verdünnungsmittel, Füllstoffe und Formulierungshilfsmittel jeder Art zu verstehen.

Als bevorzugte pharmazeutische Zubereitungen seien Tabletten, Dragees, Kapseln, Pillen, Granulate, Suppositorien, Lösungen, Suspensionen und Emulsionen, Pasten, Salben, Gele, Cremes, Lotions, Puder und Sprays genannt.

Tabletten, Dragees, Kapseln, Pillen und Granulate können den oder die Wirkstoffe neben den üblichen Trägerstoffen enthalten, wie Füll- und Streckmittel (z.B. Stärken, Milchzucker, Rohrzucker, Glukose, Mannit und Kieselsäure), Bindemittel (z.B. Carboxymethylcellulose, Alginate, Gelatine, Polyvinylpyrrolidon), Feuchthaltemittel (z.B. Glycerin), Sprengmittel (z.B. Agar-Agar, Calciumcarbonat und Natriumbicarbonat), Lösungsverzögerer (z.B. Paraffin) und Resorptionsbeschleuniger (z.B. quarternäre Ammoniumverbindungen), Adsorptionsmi-tel (z.B. Kaolin und Bentonit) und Gleitmittel (z.B. Talkum, Calcium- und Magnesiumstearat und feste Polyäthylenglykole) oder Gemische der aufgeführten Stoffe.

Die Tabletten, Dragees, Kapseln, Pillen und Granulate können mit den übliche, gegebenenfalls Opakisierungsmittel enthaltenden Überzügen und Hüllen versehen sein und auch so zusammengesetzt sein, daß sie den oder die Wirkstoffe nur oder bevorzugt in einem bestimmten Teil des Intestinaltraktes, gegebenenfalls verzögert abgeben, wobei als Einbettungsmassen z.B. Polymersubstanzen und Wachse verwendet werden können.

Der oder die Wirkstoffe können gegebenenfalls mit einem oder mehreren der oben angegebenen Trägerstoffe auch in mikroverkapselter Form vorliegen zur Erzielung eines Retardeffektes.

Suppositorien können neben dem oder den Wirkstoffen die üblichen wasserlöslichen oder wasserunlöslichen Trägerstoffe enthalten, z.B. Polyäthylenglykole, Fette, z.B. Kakaofett und höhere Ester (z.B. $C_{14}$-Alkohol mit $C_{16}$-Fettsäure) oder Gemischer dieser Stoffe.

Salben, Pasten, Cremes und Gele können neben dem oder den Wirkstoffen die üblichen Trägerstoffe enthalten (z.B. tierische und pflanzliche Fette, Wachse, Paraffine, Stärken, Tragant) oder Gemische dieser Stoffe.

Puder und Spays können neben dem oder den Wirkstoffen die üblichen Trägerstoffe enthalten (z.B. Milchzucker, Talkum, Kieselsäure, Aluminiumhydroxid) oder Gemische dieser Stoffe. Sprays können zusätzlich die üblichen Treibmittel enthalten.

Lösungen und Emulsionen können neben dem oder den Wirkstoffen die üblichen Trägerstoffe wie Lösungsmittel, Lösungsvermittler und Emulgatoren, z.B. Wasser, Äthylalkohol, Isopropylalkohol, Äthylcarbonat, Äthylacetat, Benzylalkohol, Benzylbenzoat, Propylenglykol, Öle, insbesondere Baumwollsaatöl, Erdnußöl, Maiskeimöl, Olivenöl und Sesamöl, Glycerin, Polyäthylenglykole oder Gemische dieser Stoffe enthalten.

Zur parenteralen Applikation können die Lösungen und Emulsionen auch in steriler und blutisotonischer Form vorliegen.

Suspensionen können neben dem oder den Wirkstoffen die üblichen Trägerstoffe wie flüssige Verdünnungsmittel (z.B. Wasser, Athylalkohol, Propylenglykol), Suspendiermittel (z.B. äthoxylierte Isostearylalkohole, Polyoxyäthylensorbit- und Sorbitanester, mikrokristalline Cellulose) oder Gemische dieser Stoffe enthalten.

Die genannten Formulierungsformen können auch Färbemittel, Konservierungsstoffe sowie geruchs- und geschmacksverbessernde Zusätze, z.B. Pfefferminzöl und Eukalyptusöl und Süßmittel, z.B. Saccharin enthalten.

Die therapeutisch wirksamen Verbindungen sollen in den oben aufgeführten pharmazeutischen Zubereitungen vorzugsweise in einer Konzentration von etwa 0,1 bis 99,5, vorzugsweise von etwa 0,5 bis 95 Gew.-% der Gesamtmischung, vorhanden sein.

Die oben ausgeführten pharmazeutischen Zubereitungen können außer den erfindungsgemäßen Wirkstoffen auch weitere pharmazeutische Wirkstoffe enthalten.

Die Herstellung der oben aufgeführten pharmazeutischen Zubereitungen erfolgt in üblicher Weise nach bekannten Methoden, z.B. durch Mischen des oder der Wirkstoffe mit dem oder den Trägerstoffen.

Die Wirkstoffe oder die pharmazeutischen Zubereitungen können lokal, oral, parenteral, intraperitoneal und/oder rectal, vorzugsweise parenteral, appliziert werden.

Im allgemeinen ist es in der Humanmedizin als vorteilhaft anzusehen, den oder die erfindungsgemäßen Wirkstoffe in Gesamtmengen von etwa 20 bis 800, vorzugsweise 100 bis 500 mg je 24 Stunden, gegebenenfalls in Form mehrerer Einzelgaben zur Erzielung der gewünschten Ergebnisse, zu verabreichen. Eine Einzelgabe enthält den oder die erfindungsgemäßen Wirkstoffe, vorzugsweise in Mengen von etwa 10 bis etwa 300, insbesondere 50 bis 200 mg/Dosis. Es kann jedoch erforderlich sein, von den genannten Dosierungen abzuweichen und zwar in Abhängigkeit von der Art und dem Körpergewicht des zu behandelnden Objekts, der Art und der Schwere der Erkrankung, der Art der Zubereitung und der Applikation des Arzneimittels sowie dem Zeitraum bzw. Intervall, innerhalb welchem die Verabreichung erfolgt. So kann es in einigen Fällen ausreichend sein, mit weniger als der obengenannten Menge Wirkstoff auszukommen, während in anderen Fällen die oben angeführte Wirkstoffmenge überschritten werden muß. Die Festlegung der jeweils erforderlichen optimalen Dosierung und Applikationsart der Wirkstoffe kann durch jeden Fachmann aufgrund seines Fachwissens leicht erfolgen.

## Herstellungsbeispiele

### Beispiel 1

S-Methyl-3-methylsulfinylpropionthioat

$CH_3SO—CH_2—CH_2—COSCH_3$

3 g (0,02 Mol) S-Methyl-3-methylthiopropionthioat werden in einem Gemisch aus 200 ml Wasser und 160 ml Aceton gelöst, mit 3 Tropfen Eisessig und innerhalb von 5 1/2 Stunden tropfenweise mit einer Lösung von 2,83 ml 30%igem Wasserstoffperoxid versetzt und über Nacht aufbewahrt. Dann wird die Reaktionslösungs im Vakuum eingedampft und der ölige Rückstand sieben Tage bei 5°C aufbewahrt. Der zum Teil kristalline Brei wird in Äthylacetat aufgenommen und an Kieselgel chromatographiert. In dieser Weise werden 0,75 g (22,6% der Theorie) S-Methyl-3-methylsulfinylpropionthioat als Öl erhalten.

$C_5H_{10}O_2S_2$ (166,3)

| | | | |
|---|---|---|---|
| berechnet: | C 36,12%, | H 6,06%, | S 38.57%; |
| gefunden: | C 36,06%, | H 5,99%, | S 38,54%. |

### Beispiel 2

S-Methyl-3-methylsulfonylpropionthioat

$CH_3SO_2CH_2CH_2COSCH_3$

Die Verbindung wird als schneller wandernde Fraktion bei der Chromatographie bei Beispiel 1 erhalten.

Fp. 72 bis 73°C; Ausbeute: 0,5 g (13% der Theorie).

$C_5H_{10}O_3S_2$ (182,267)

| | | | |
|---|---|---|---|
| berechnet: | C 32,95%, | H 5,53%, | S 35,18%; |
| gefunden: | C 32,99%, | H 5,51%, | S 35,18%. |

### Beispiel 3

S-Methyl-3-methoxypropionthioat

$CH_3OCH_2—CH_2COSCH_3$

Eine Lösung von 20,8 g (0,2 Mol) 3-Methoxypropionsäure in 60 ml Dichlormethan wird mit einer Lösung von 41,2 g (0,2 Mol) Dicyclohexylcarbodiimid in 40 ml Dichlormethan und nach Abkühlen auf —10°C mit 12 ml (0,22 Mol) Methanthiol (vorgekühlt mit Aceton/$CO_2$) versetzt. Nach 15-stündigem Stehen bei Raumtemperatur wird überschüssiges Dicyclohexylcarbodiimid durch Zugabe von 2 ml 50%iger Essigsäure vernichtet, die Reaktionslösung gerührt und kurze Zeit später vom ausgefallenen Harnstoff abfiltriert. Das Filtrat wird mit Wasser gewaschen, getrocknet und im Vakuum destilliert.

Es werden 9,1 g (34% der Theorie) an S-Methyl-3-methoxypropionthioat als gelbliches Öl vom $Kp_{12-13}$ 65 bis 70°C erhalten.

$C_5H_{10}O_2S$ (134,201)

berechnet: C 44,75%, H 7,51%, S 23,89%;
gefunden: C 44,87%, H 7,46%, S 23,73%.

### Beispiel 4

Äthyl-3-methylthiopropionat

Eine Mischung aus 20 g (0.2 Mol) Äthylacrylat in 100 ccm Tetrahydrofuran (0,25 Mol) und 2,35 g (21 m Mol) Kaliumtert.-butylat wird 8 Tage bei Raumtemperatur und anschließend 4 Stunden bei 50°C in einem Laborautoklaven aufbewahrt. Das Reaktionsgemisch wird filtriert, das Filtrat im Vakuum eingedampft und das verbleibende leichtbeweglich Öl destilliert.

$Kp._{12}$ 84—86°C; Ausbeute: 24,1 g (81,5% der Theorie).
(Lit. $Kp._{18}$ 88°C)

## Allgemeine Herstellungsvorschrift für S-Alkyl-3-alkylthiopropionthioate

### Beispiel 5

S-Methyl-3-methylthiopropionthioat

$CH_3SCH_2CH_2COSCH_3$

Eine Lösung von 8 g (0,2 Mol) NaOH in 160 ccm Wasser wird auf —10°C gekühlt und mit einer in Aceton/Trockeneis gekühlten 25 ccm Ampulle (0,466 Mol) Methylmercaptan und anschließend portionsweise mit 18,1 g = 15,8 ccm (0,2 Mol) Acrylsäurechlorid versetzt. Nach der letzten Zugabe rührt man 2 Stunden bei Raumtemperatur und läßt dann über Nacht stehen.

Das ausgeschiedene Öl wird abgetrennt, die wäßrige Phase mit Äther ausgeschüttelt und Öl- und Ätherphase vereinigt, über $Na_2SO_4$ getrocknet und fraktioniert.

$Kp._{20}$ 114—117°C; Ausbeute: 24,3 g (81% der Theorie), schwach gelbliches Öl.

$C_5H_{10}OS_2$ (150,267)

berechnet: C 39,97%, H 6,71%, S 42,68%;
gefunden: C 40,20%, H 6,63%, S 42,50%.

In ähnlicher Weise werden hergestellt:

### Beispiel 6

S-Äthyl-3-äthylthiopropionthioat

$C_2H_5SCH_2CH_2COSC_2H_5$

$Kp._{55}$ 157—160°C; Ausbeute: 67,4% der Theorie.

$C_7H_{14}OS$ (178,3)

berechnet: C 47,15%, H 7,91%, S 35,96%;
gefunden: C 47,30%, H 7,85%, S 35,91%.

### Beispiel 7

S-Propyl-3-propylthiopropionthioat

$C_3H_7SCH_2CH_2COSC_3H_7$

$Kp._{0,08}$ 78—80°C; Ausbeute: 78% der Theorie.

$C_9H_{18}OS_2$ (206,4)

berechnet: C 52,38%, H 8,79%, S 31,08%;
gefunden: C 52,47%, H 8,83%, S 30,97%.

## Beispiel 8

S-Isopropyl-3-isopropylthiopropionthioat

$(CH_3)_2CHSCH_2CH_2COSCH(CH_3)_2$

$Kp._{0.15}$ 82—83°C; Ausbeute: 70% der Theorie.

$C_9H_{18}OS_2$ (206,4)

berechnet: C 52,38%, H 8,79%, S 31,08%;
gefunden: C 52.36%, H 8,66%, S 30,95%.

## Beispiel 9

S-Butyl-3-butylthiopropionthioat

$C_4H_9SCH_2CH_2COSC_4H_9$

$Kp._{0.15}$ 95—102°C; Ausbeute: 80% der Theorie.

$C_{11}H_{22}OS_2$ (234,4)

berechnet: C 56,36%, H 9,46%, S 27,36%;
gefunden: C 56,40%, H 9,44%, S 27,48%.

## Beispiel 10

S-sek.Butyl-3-sek.butylthiopropionthioat

$CH_3CH_2CH(CH_3)SCH_2CH_2COSCH(CH_3)CH_2CH_3$

$Kp._{0,2}$ 101—105°C; Ausbeute: 69% der Theorie.

$C_{11}H_{22}OS_2$ (234,4)

berechnet: C 56,36%, H 9,46%, S 27,36%;
gefunden: C 56,30%, H 9,46%, S 27,35%.

## Beispiel 11

S-tert.Butyl-3-tert.butylthiopropionthioat

$(CH_3)_3CSCH_2CH_2COSC(CH_3)_3$

$Kp._{28}$ 160—162°C (Lit. $Kp_{11}$ 135—136°); Ausbeute: 50% der Theorie.

$C_{11}H_{22}OS_2$ (234,4)

berechnet; C 56,36%, H 9,46%, S 27,36%;
gefunden: C 56,48%, H 9,35%, S 27,14%.

## Beispiel 12

S-Isobutyl-3-isobutylthiopropionthioat

$CH_3CH(CH_3)CH_2SCH_2CH_2COSCH_2CH(CH_3)CH_3$

$Kp._{0,2}$ 107—110°C; Ausbeute: 64% der Theorie.

$C_{11}H_{22}OS_2$ (234,4)

berechnet: C 56,36%, H 9,46%, S 27,36%;
gefunden: C 56,30%, H 9,41%, S 27,40%.

Oxidation der vorstehenden S-Alkyl-3-alkylthiopropionthioate

Methode A: in Eisessig mit 1 Mol Wasserstoffperoxid.

Beispiel 13

S-Butyl-3-butylsulfinylpropionthioat

$C_4H_9SOCH_2CH_2CO—SC_4H_9$

9,37 g (40 m Mol) S-Butyl-3-butylthiopropionthioat werden in 9 ccm Eisessig gelöst und unter Rühren und Kühlen im Eisbad tropfenweise mit 4 ccm (40 m Mol) 30% Wasserstoffperoxid versetzt. Nach Erwärmung auf Raumtemperatur läßt man 2 Stunden reagieren, verdünnt dann mit Wasser und extrahiert anschließend das Produkt mit Methylenchlorid. Nach Verdampfen des Lösungsmittels im Vakuum wird an Kieselgel chromatographiert. Durch Waschen mit Äther werden geringe Mengen S-Butyl-3-butylthiopropionthioat und S-Butyl-3-butylsulfonylpropionthioat abgetrennt. Anschließende Elution mit Methylenchlorid und Verdampfen des Lösungsmittels im Vakuum ergibt 5,75 g (57,5% der Theorie) öliges S-Butyl-3-butylsulfinylpropionthioat.

$C_{11}H_{22}O_2S_2$ (250,4)

berechnet: C 52,76%, H 8,85%, S 25,61%;
gefunden: C 52,99%, H 8,48%, S 24,32%.

In ähnlicher Weise werden hergestellt:

Beispiel 14

S-sek.-Butyl-3-sek.-butylsulfinylpropionthioat

$CH_3CH_2CH(CH_3)SOCH_2CH_2COSCH(CH_3)CH_2CH_3$

Ausbeute: 89% der Theorie; Öl.

$C_{11}H_{22}O_2S_2$ (250,4)

berechnet: C 52,76%, H 8,85%, S 25,61%;
gefunden: C 52,84%, H 9,89%, S 25,63%.

Beispiel 15

S-Isopropyl-3-isopropylsulfinylpropionthioat

$(CH_3)_2CHSOCH_2CH_2COSCH(CH_3)_2$

Ausbeute: 62,5% der Theorie; Öl.

$C_9H_{18}O_2S_2$ (222,4)

berechnet: C 48,61%, H 8,16%, S 28,84%;
gefunden: C 48,84%, H 8,00%, S 28,84%.

Beispiel 16

S-Propyl-3-propylsulfinylpropionthioat

$C_3H_7SOCH_2CH_2COSC_3H_7$

Asbeute: 56,3% der Theorie; Öl.

$C_9H_{18}O_2S_2$ (222,4)

berechnet: C 48,61%, H 8,16%, S 28,84%;
gefunden; C 48,58%, H 8,26%, S 28,75%.

Beispiel 17

S-Äthyl-3-äthylsulfinylpropionthioat

$C_2H_5SOCH_2CH_2COSC_2H_5$

Fp. 35,5—36°C; Ausbeute: 55,4% der Theorie.

$C_7H_{14}O_2S_2$ (194,3)

berechnet: C 43,27%, H 7,26%, S 33,00%;
gefunden: C 43,15%, H 7,30%, S 32,97%.

Beispiel 18

S-tert.-Butyl-3-tert.-butylsulfinylpropionthioat

$(CH_3)_3CSOCH_2CH_2COSC(CH_3)_3$

Fp. 72,5—73,5°C; Ausbeute: 61,9% der Theorie.

$C_{11}H_{22}O_2S_2$ (250,4)

berechnet: C 52,76%, H 8,85%, S 25,61%;
gefunden: C 52,85%, H 8,70%, S 25,51%.

Methode B: in Eisessig mit 2 Mol Wasserstoffperoxid, Reaktionszeit 3—4 Tage.

Beispiel 19

S-Äthyl-3-äthylsulfinylpropionthioat und S-Äthyl-3-äthylsulfonylpropionthioat

$C_2H_5SO_2CH_2CH_2COSC_2H_5$

7,13 g (40 m Mol) S-Äthyl-3-äthylthiopropionthioat werden in 5 ccm Eisessig gelöst und unter Rühren und Kühlen im Eisbad tropfenweise mit 8 ccm (80 m Mol) 30% Wasserstoffperoxid versetzt. Man läßt die Reaktionslösung drei Tage bei Raumtemperatur reagieren, verdünnt mit Wasser, extrahiert das Reaktionsgemisch mit Methylenchlorid und chromatographiert das nach Verdampfen des Lösungsmittels verbleibende Öl an Kieselgel.

Elution mit Äther ergibt 1,5 g (17,8% der Theorie) S-Äthyl-3-äthylsulfonyl-propionthioat vom Fp. 35—36°C.

$C_5H_{10}O_3S_2$ (182,3)

berechnet: C 39,98%, H 6,71%, S 30,49%;
gefunden: C 40,17%, H 6,71%, S 30,40%.

Durch anschließende Elution mit Aceton werden 2,8 g (36,1% der Theorie) S-Äthyl-3-äthylsulfinylpropionthioat vom Fp. 35,5—36°C erhalten.

In ähnlicher Weise werden hergestellt:

Beispiel 20

S-sek.-Butyl-3-sek.-butylsulfonylpropionthioat

$CH_3CH_2CH(CH_3)SO_2CH_2CH_2COSCH(CH_3)CH_2CH_3$

aus S-sek.-Butyl-3-sek.-butylthiopropionthioat.

Ausbeute: 31,1% der Theorie; Öl.

$C_{11}H_{22}O_3S_2$ (266,4)

berechnet: C 49,59%, H 8,32%, S 24,07%;
gefunden: C 50,35%, H 8,29%, S 23,71%.

Daneben: S-sek.-Butyl-3-sek.-butylsulfinylpropionthioat

Ausbeute: 30% der Theorie.

Beispiel 21

S-Isobutyl-3-isobutylsulfonylpropionthioat

$(CH_3)_2CHCH_2SO_2CH_2CH_2COSCH_2CH(CH_3)_2$

aus S-Isobutyl-3-isobutylthiopropionthioat.

Fp. 43—44°C; Ausbeute: 20% der Theorie.

$C_{11}H_{22}O_3S_2$ (266,4)

berechnet: C 49,59%; H 9,32%, S 24,07%;
gefunden: C 49,70%, H 8,29%, S 24,01%.

Daneben: S-Isobutyl-3-isobutylsulfinylpropionthioat

Ausbeute: 30% der Theorie; wachsige Substanz.

$C_{11}H_{22}O_2S_2$ (250,4)

berechnet: C 52,76%, H 8,85%, S 25,61%;
gefunden: C 52,94%, H 8,91%, S 25,86%.

Beispiel 22

S-tert.-Butyl-3-tert.-butylsulfonylpropionthioat

$(CH_3)_3CSO_2CH_2CH_2COSC(CH_3)_3$

aus tert.-Butyl-3-ter.-butylthiopropionthioat.

Fp. 98—99°C; Ausbeute: 22,6% der Theorie.

$C_{11}H_{22}O_3S_2$ (266,4)

berechnet: C 49,59%, H 8,32%, S 24,07%;
gefunden: C 49,47%, H 8,18%, S 24,24%.

Daneben: S-tert.-Butyl-3-tert.-butylsulfinylpropionthioat

Fp. 73—74°C; Ausbeute: 34% der Theorie.

Methode C: in Eisessig mit 2 Mol Wasserstoffperoxid; Reaktionszeit 10—14 Tage.

Beispiel 23

S-sek.-Butyl-3-sek.-butylsulfonylpropionthioat

9,37 g (40 m Mol) S-sek.-Butyl-3-sek.-butylthiopropionthioat werden in 17 ccm Eisessig gelöst und unter Rühren und Kühlen im Eisbad tropfenweise mit 8 ccm (~80 m Mol) 30% Wasserstoffperoxid versetzt. Man läßt 14 Tage bei Raumtemperatur reagieren, verdünnt mit Wasser, extrahiert das Reaktionsgemisch mit Methylenchlorid und chromatographiert den öligen Eindampfrückstand des Extraktes an Kieselgel.

Durch Elution mit Diisopropyläther werden 2,35 g (22,2% der Theorie) S-sek.-Butyl-3-sek.-butylsulfonylpropionthioat erhalten.

An ähnlicher Weise werden hergestellt:

Beispiel 24

S-Butyl-3-butylsulfonylpropionthioat

aus S-Butyl-3-butylthiopropionthioat.

Fp. 46—47°C; Ausbeute: 10,4% der Theorie.

$C_{11}H_{22}O_3S_2$ (266,4)

| | | | |
|---|---|---|---|
| berechnet: | C 49,59%, | H 8,32%, | S 24,07%; |
| gefunden: | C 49,82%, | J 8,38%, | S 23,87%. |

Beispiel 25

S-Isobutyl-3-isobutylsulfonylpropionthioat

aus S-Isobutyl-3-isobutylthiopropionthioat.

Fp. 42—43°C; Ausbeute: 6,1% der Theorie.

Methode D: in wäßrigem Aceton mit 2 Mol Wasserstoffperoxid.

Beispiel 26

S-Isopropyl-3-isopropylsulfonylpropionthioat

$(CH_3)_2CHSO_2CH_2CH_2COSCH(CH_3)_2$

und S-Isopropyl-3-isopropylsulfinylpropionthioat

4,12 g (20 m Mol) S-Isopropyl-3-isopropylthiopropionthioat werden in einem Gemisch aus 20 ccm Wasser und 60 ccm Aceton gelöst und nach Kühlen auf 0°C mit einigen Tropfen Eisessig und tropfenweise unter Rühren mit 4 ccm (40 m Mol) 30% Wasserstoffperoxid versetzt. Das Reaktionsgemisch wird 8 Tage bei Raumtemperatur aufbewahrt, zur Zerstörung überschüssigen Wasserstoffperoxids mit einer Spaltspitze Platinmohr behandelt, filtriert, im Vakuum eingedampft und an Kieselgel chromatographiert.

Durch Elution mit Äther werden 1,9 g (39,9% der Theorie) S-Isopropyl-3-isopropylsulfonylpropionthioat vom Fp. 45—47°C erhalten.

$C_9H_{18}O_3S_2$ (238,4)

| | | | |
|---|---|---|---|
| berechnet: | C 45,34%, | H 7,62%, | S 26,90%; |
| gefunden: | C 45,53%, | H 7,56%, | S 26,80%. |

Anschließende Elution mit Aceton ergibt 1,2 g (27% der Theorie) öliges S-Isopropyl-3-isopropylsulfinylpropionthioat.

In ähnlicher Weise werden hergestellt:

Beispiel 27

S-Propyl-3-propylsulfonylpropionthioat

$C_3H_7SO_2CH_2COSC_3H_7$

aus S-Propyl-3-propylthiopropionthioat.

Fp. 55—56°C; Ausbeute: 18,5% der Theorie.

$C_8H_{18}O_3S_2$ (238,4)

| | | | |
|---|---|---|---|
| berechnet: | C 45,34%, | H 7,62%, | S 26,90%; |
| gefunden: | C 45,63%, | H 7,59%, | S 26,91%. |

**Patentansprüche**

1. Arzneimittel enthaltend mindestens einen schwefelhaltigen Ester der allgemeinen Formel I

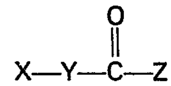

(I)

in welcher
X für ein Wasserstoffatom, eine Alkylgruppe, eine Alkoxygruppe, eine Alkylthiogruppe, eine Alkylsulfinyl- oder Alkylsulfonylgruppe oder für eine Hydroxy- oder eine Mercaptogruppe steht,
Y für die Gruppierung

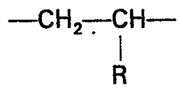 oder 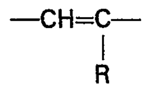

wobei R für ein Wasserstoffatom oder eine Alkylgruppe steht, und
Z für eine Alkylthiogruppe steht.

2. Antiphlogistisch wirkendes Arzneimittel gemäß Anspruch 1.

3. Verfahren zur Herstellung von Arzneimitteln, dadurch gekennzeichnet, daß man schwefelhaltige Ester gemäß Formel I in Anspruch 1, gegebenenfalls unter Zusatz von inerten pharmazeutisch unbedenklichen Hilfs- und Trägerstoffen, in eine geeignete Applikationsform überführt.

4. Arzeneimittel enthaltend mindestens einen schwefelhaltigen Ester der allgemeinen Formel I

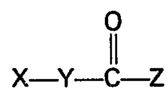

(I)

in welcher
X für Wasserstoff, Alkyl, Alkoxy, Alkylthio, Alkylsulfinyl, Alkylsulfonyl oder eine Mercaptogruppe steht, wobei die genannten Alkyl- und Alkoxygruppen jeweils 1 bis 4 Kohlenstoffatome enthalten,
Y für die Gruppierung

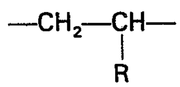 oder 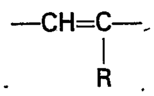,

steht, wobei R für Wasserstoff oder Alkyl mit 1 bis 4 Kohlenstoffatomen steht und
Z für Alkylthio mit 1 bis 4 Kohlenstoffatomen in der Alkylgruppe steht.

**Claims**

1. A medicament containing at least one sulphur-containing ester of the general formula I

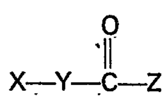

(I)

in which
X represents a hydrogen atom, an alkyl group, an alkoxy group, an alkylthio group, an alkylsulphinyl or alkylsulphonyl group or a hydroxyl or mercapto group,
Y represents the grouping

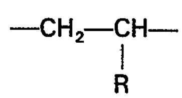 or 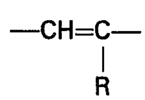

in which R represents a hydrogen atom or an alkyl group, and
Z represents an alkylthio group.

2. A medicament having an antiphlogistic action, according to claim 1.

3. Process for the production of medicaments, characterised in that sulphur-containing esters according to formula I in claim 1 are converted into a suitable application form, optionally with the addition of inert, pharmaceutically acceptable auxiliaries and excipients.

4. Medicaments containing at least one sulphur-containing ester of the general formula I

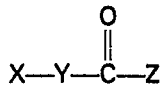 (I)

in which

X represents hydrogen, alkyl, alkoxy, alkylthio, alkylsulphinyl, alkylsulphonyl or a mercapto group, the named alkyl and alkoxy groups containing in each case 1 to 4 carbon atoms,

Y represents the grouping

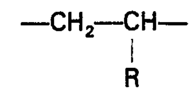 or 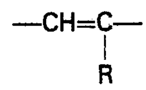

in which R represents hydrogen or alkyl with 1 to 4 carbon atoms and

Z represents alkylthio with 1 to 4 carbon atoms in the alkyl group.

**Revendications**

1. Médicament contenant au moins un ester sulfuré de formule générale I

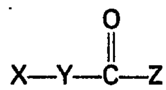 (I)

dans laquelle

X représente un atome d'hydrogène, un groupe alcoyle, un groupe alcoxy, un groupe alcoylthio, un groupe alcoylsulfinyle ou alcoylsulfonyle, ou un groupe hydroxy ou mercapto,

Y représente le groupement

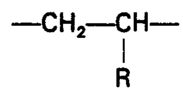 ou 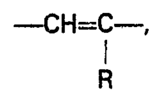,

où R représente un atome d'hydrogène ou un groupe alcoyle, et

Z représente un groupe alcoylthio.

2. Médicament à activité antiphlogistique selon la revendication 1.

3. Procédé de préparation de médicaments, caractérisé en ce qu'on convertit un ester sulfuré, selon la formule I donnée à la revendication 1, éventuellement avec addition d'auxiliaires et de véhicules inertes pharmaceutiquement inoffensifs, en une forme d'application appropriée.

4. Médicament contenant au moins un ester sulfuré de formule générale I

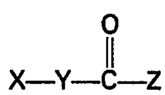 (I)

dans laquelle

X représente de l'hydrogène, alcoyle, alcoxy, alcoylthio, alcoylsulfinyle, alcoylsulfonyle ou un groupe mercapto, les groupes alcoyle et alcoxy cités ayant chaque fois 1 à 4 atomes de carbone,

Y représente le groupement

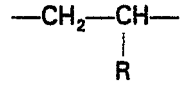 ou 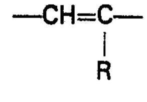

R représentant de l'hydrogène ou an alcoyle avant 1 à 4 atome de carbone et

Z représente un alcoylthio ayant 1 à 4 atomes de carbone dans le groupe alcoyle.